# EUROPEAN PATENT APPLICATION

(11) **EP 2 072 625 A1**
(43) Date of publication of application: **24.06.2009**
(21) Application number: 07150177.9
(22) Date of filing: 19.12.2007
(51) Int. Cl.: C12Q 1/68

(54) **Means and methods for typing a cell isolate of an individual suffering from a psychiatric disorder or at risk of suffering there from**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Hatzmann, Martin

(57) **Abstract**

The invention relates to methods and means for typing a cell isolate of an individual suffering from a pychiatric disorder, or at risk for suffering there from, by determining RNA expression levels of a set of genes. The invention further relates to primers and probes for determining said RNA expression levels and uses thereof.

## Description

The invention relates to the field of medicine and to the field of diagnostics. The invention in particular relates to means and methods for typing cells and cell isolates from individuals suffering from, or at risk of suffering from, a psychiatric disorder, particularly a depressive disorder.

The invention is examplied herein below predominantly for depressive disorders, however, this does not mean that the invention is limited thereto. The means and methods of the invention are suited for all psychiatric disorders, depressive disorders are nevertheless preferred. Depressive and anxiety disorders, have a high lifetime prevalence (16% and 10%, respectively) and frequently run a chronic course. There is considerable co-morbidity between depressive and anxiety disorders. They have many symptoms in common, similar pathogenic mechanisms are proposed and both disorders can be treated successfully with similar antidepressants. The pathogenesis of depressive and anxiety disorders is largely unknown but it is clear that genetic vulnerability and environmental risk factors are both important. At least two meta-analyses (Sullivan et al., 2000; Hettema et al., 2001) suggest that familial aggregation for depressive and anxiety disorders are due to genetic effects (heritability: depression 37%, panic disorder 43%, generalized anxiety disorder 32%), with a minimal contribution of environmental effects shared by family members, but substantial individual-specific environmental effects.

Depression has a large impact on human health (morbidity and mortality) and society in general and will be the second most important medical disorder by the year 2020, according to the World Health Organization. The three brain systems that are most strongly implicated in the pathogenesis of depression are: (i) the midbrain serotonin (5-HT) system; (ii) the medial prefrontal cortex (mPFC); and (iii) the
hypothalamopituitary-adrenal (HPA) axis. Midbrain serotonergic neurons in the raphe nuclei participate in many physiological functions and are considered to be the cellular target of serotonin-specific reuptake inhibitors (SSRIs), the main class of drugs used to treat psychiatric illnesses. Their activity is controlled by 5-HT1A autoreceptors and by input from many brain areas, including the mPFC (see Hajós et al., 1998; Peyron et al., 1995). A disturbed 5-HT transmission of the raphe nuclei to their targets forms a plausible explanation for depression since SSRI's successfully normalise mood in many patients, whereas depletion of serotonin precursor tryptophan results in acute recurrence of depression (Artigas et al., 1996; Stahl, 2000; Blier and Bergeron, 1998; Haddjeri et al., 1998). In animal models, the 5-HT1A receptor was shown to act during development to establish normal anxiety-like behaviour in the adult (Gross et al., 2002). Relevance to humans is demonstrated by decreased binding of 5-HT1A tracers (Drevets et al., 2000; Sargent et al., 2000) and increased 5-HT1A autoreceptor density in the RN of depressed patients (Stockmeier et al., 1998). Polymorphisms of the serotonin transporter gene have also been associated with depression symptoms (Mann et al., 2000) and the occurrence of depression after stressful life-events (Caspi et al., 2003) or tryptophane depletion (Neumeister et al., 2002).

The prefrontal cortex regulates cognitive and associative functions and is involved in planning and execution of complex tasks (see Fuster, 1997). Depressed patients show hypoperfusion in mood related areas of the PFC (Drevets et al., 1997), which correlates with depression severity and normalises during treatment with SSRI (Soares and Mann, 1997). The mPFC is one of the few forebrain areas that projects densely to the serotonergic RN (Hajós et al., 1998; Peyron et al., 1995) and electrical stimulation of the mPFC indeed modulates the activity of 5-HT neurons (Hajós et al., 1998; Celada et al., 2001). In turn, the mPFC is densely innervated by serotonergic RN afferents (Azmitia and Segal, 1978).

Especially layer V pyramidal neurons express 5-HT1A and 5-HT2A receptors (Pompeiano et al., 1994; Kia et al., 1996) and GABAergic interneurons express 5-HT2A and 5-HT3 receptors (see Jakab and Goldman-Rakic, 2000). The precise impact of 5-HT receptor activation in the mPFC microcircuits is currently unknown and may hold important clues to the understanding of depression. Hyperactivity of the HPA-axis is found in about 50% of depression patients. Successful antidepressive treatment often normalizes the HPA-axis (Inder et al., 2001) and corticotrophin releasing hormone (CRH)1-antagonists have antidepressant effects (Kunzel et al., 2003). Disturbed 5-HT transmission influences hypothalamic CRH producing neurons and the ensuing ACTH release from the pituitary and cortisol from the adrenal in animals and humans (Jorgensen et al., 2003). Furthermore, increased CRH gene expression in these hypothalamic targets of the serotonin system was observed in post mortem material of depressed patients (Raadsheer et al., 1995). Whether disturbed HPA axis activity is secondary to a disturbed 5-HT system or constitutes its own source of pathology remains an open question (see Kagamiishi et al., 2003; Montgomery et al., 2001; Oshima et al., 2003; Summers et al., 2003). In either case, the HPA axis provides a non-invasive read-out parameter, cortisol, which can be assessed in animals and in large scale human studies, by means of provocation tests (e.g. dexamethasone suppression test) and non-invasive salivary sampling (Kirschbaum and Hellhammer, 1989). Taken together, the evidence outlined above clearly identifies the raphe serotonin system, the mPFC and the HPA axis as central systems in the pathogenesis of the depression spectrum and emphasizes the evident interplay between the three systems.

Although much progress has been made in the field of depression, there is still much to be learned. Depression and anxiety disorders frequently run a chronic course, with a number of negative health care consequences, including increased medical consumption, disability, somatic morbidity and mortality. Given an extremely variable natural history, the most viable route for prevention appears to be to design ways to detect those at risk for an unfavourable prognosis in an early stage, tailoring interventions to the projected prognosis. A sobering finding, common to most psychiatric disorders, is that, until now, not one single determinant explains more than 15% of the aetiology of either disorder (McGuire and Troisi, 1998). Factors that have been identified are mostly very common, implying that they do not carry very high relative or absolute risks for incidence. Moreover, many risk factors (such as multiple loss) are not open to intervention. This severely limits the scope for primary and secondary prevention. Major depression is by far the most widely studied condition, concerning determinants of prognosis (for review, see Spijker et al., 2002). The predictive power of clinical factors, such as co-morbidity or duration and severity of earlier episodes, although limited, is hopeful, as these factors can be assessed in routine clinical work. However, these factors do not *explain* the wide variation in the prognosis. It is highly likely that underlying (molecular) biological factors determine both the clinical features of index episodes and (in interaction with environmental factors) their subsequent course. Moreover, individuals with similar depression-like symptoms can have very different clinical outcomes later in life. In fact many individuals suffer from depression-like symptoms at some point in their life but these are temporary and full recovery is possible without reverting to medication treatment. It is, at present, difficult to distinguish between groups that will recover without medication treatment and groups that do not recover without medication. This makes it difficult to determine the best treatment plan for individuals that present themselves with depression-like symptoms. Moreover, after a first depressive episode, about 50% of the patients will experience a recurrence of depression within a year. Therefore, physicians advise the patient according to protocol, to stay on antidepressant medication for six month after remission. However, most patients do not comply to this advice because it is difficult to take medication at a time there is no burden of disease, while adverse effects may be present. Furthermore, with our present knowledge we can not predict whether a particular patient belongs to the 50% in whom depression would indeed reoccur, when of medication. Finally, at present physicians can not predict which type of antidepressant (e.g. noradrenergic or serotonergic) will be effective for a particular patient, and have minimal adverse effects. The present invention relates to means and methods for typing and predicting: 1) which individuals with
depression-like symptoms will become depressed when of medication; 2) which individuals with a depressive disorder will suffer from reoccurrence after premature termination of prophylactic antidepressant treatment; and 3) which patients benefit from what type of antidepressants, in terms of optimal effect and minimal adverse effect.

In particular the invention provides a method for typing a cell isolate of an individual suffering from a psychiatric disorder, or at risk of suffering there from, the method comprising providing an RNA sample from a cell isolate from said individual, determining RNA levels for a set of genes in said RNA sample, wherein said set of genes comprises at least two of the genes listed in Table 1, and typing said isolate on the basis of the levels of RNA determined for said set of genes.

Part of the biological changes described above is probably caused by genetic polymorphisms (Flint at al., 1995; Holsboer et al., 1995). It is generally expected that in the following years a large number of polymorphisms will be discovered in candidate genes that code for proteins, which are already known to be involved in the pathogenesis of a psychiatric disease such as, for example, a depression (Merikangas, 2002). Twin studies have shown that depression is at least partly genetically determined, whereby genetic polymorphisms underlie biological factors which, in interaction with environmental factors, determine the course of a psychiatric disease such as a depressive disorder. A method according to the invention allows typing of a cell isolate from an individual based on RNA levels of a set of genes in said cell isolate. Without being bound by theory it is believed that at least some of the differences in gene expression detected using a method of the invention are due to differentially expressed polymorphic genes. These genes are typically associated with their most proximal gene product (i.e. gene-expression-and protein profiles). Polymorphic differences underlying a psychiatric disease such as a depressive disorder are at least to some extent reflected in differences in gene expression patterns present in a cell isolate. Furthermore, cells of a patient have been exposed to factors related to the disease (e.g. elevated cortisol levels and probably various other as yet unknown factors), which can cause differences in gene expression status of cells between healthy controls and patients. These differences can be visualized by analyzing gene expression such as RNA levels. If required, cells can be contacted with a stimulus for enhancement of differences in gene expression.

Thus, polymorphic genes and the internal and external (with or without stimulus) cell environment provide a unique combination resulting in differences in gene expression patterns between patients and controls.

A preferred method according to the invention further comprises providing an RNA sample from said cell isolate after contacting said cell isolate with a stimulus, Said stimulus can be selected from cytokines such as stem cell factor, colony-stimulating factor, hepatocyte growth factor, interferon, leukemia inhibitory factor, transforming growth factor beta, tumor necrosis factor alpha, and interleukins; lipopolysaccharides (LPS); neurotransmitters such as acetylcholine, norepinephrine, dopamine, serotonin, and gamma aminobutyric acid; and hormones such as vasopressin, thyroid hormone, oestradiol, progesteron, testosteron, glucocorticoid and dehydroepiandosteron. Preferred stimuli are interleukin (IL) 6 and IL 10, TNF alpha; glucocorticoid, and LPS. A particularly preferred stimulus is provided by LPS, which appeared to be a potent stimulus especially for blood cells.

Preferably, said typing in a method according to the invention is based on a comparison with a reference. In one embodiment, said reference comprises the corresponding RNA levels for said set of genes in an RNA sample from a cell isolate from an individual or a group of individuals not suffering and/or not at risk of suffering from said psychiatric disorder. Alternatively, said reference comprises the corresponding RNA levels for said set of genes in an RNA sample from a cell isolate from an individual or a group of individuals suffering from said psychiatric disorder. In a preferred embodiment, said reference sample comprises both samples from an individual or a group of individuals not suffering and/or not at risk of suffering from said psychiatric disorder and from an individual or a group of individuals suffering from said psychiatric disorder. It is preferred that said group of individuals comprises at least two individuals, more preferred at least three individuals, more preferred at least four individuals, more preferred at least five individuals, more preferred at least ten individuals. A preferred reference comprises RNA levels determined for said set of genes in a cell isolate prior to and/or after contacting said cell isolate with a stimulus.

In a preferred embodiment, said RNA levels from a reference is stored on an electronic storage device, such as, but not limited to, a computer or a server. Said reference can be addressed to compare the determined RNA levels of an individual suffering from a psychiatric disorder, or at risk of suffering there from, with said reference. Said comparison preferably provides a resemblance score, which is a measure for the similarity of the determined RNA levels of said individual with the corresponding levels in said reference. An arbitrarily determined threshold can be provided to classify an individual into a group with a high resemblance score as compared to said reference, and a group with a low resemblance score as compared to said reference.

The invention further provides a method for typing a cell isolate of an individual suffering from a psychiatric disorder, or suspected of suffering there from, the method comprising providing a first RNA sample from a cell isolate from said individual, providing a second RNA sample from said cell isolate after contacting said cell isolate with a stimulus, determining RNA levels for a set of genes in said first and second RNA sample, wherein said set of genes comprises at least two of the genes listed in Table 1, and typing said isolate on the basis of the levels of RNA determined for said set of genes.

Preferably, said typing is based on the levels of RNA as determined in said RNA sample provided after contacting the cell isolate with a stimulus, or whereby said typing is based on the ratio of RNA levels determined in said RNA samples provided from a cell isolate prior to an after contacting with a stimulus. It was found by the inventors that typing based on levels of RNA determined for said set of genes after contacting cells with a stimulus, or based on the ratio of RNA levels before and after contacting said cell isolate with a stimulus, provides a more robust typing whereby more information about the disease state of the individual is captured.

A psychiatric disorder comprises a clinical disorder, a mental disorder, a psychotic disorder such as schizophrenia, a depressive disorder, a substance-related disorder such as an alcohol-related disorder or a
sedative-related disorder, a somatoform disorder, a factitious disorder, a dissociative disorder, or a personality disorder. A preferred psychiatric disorder comprises a depressive disorder.

The term cell isolate refers to a tissue sample comprising cells from an individual. Preferably, said tissue sample is a sample that can easily be isolated from said individual, including but not limited to a biopsy such as for example a skin biopsy comprising keratinocytes, a buccal swap comprising mucosa cells, and blood comprising blood cells. A preferred tissue sample for use in a method of the invention is provided by whole blood comprising blood cells such as peripheral mononuclear blood cells (PBMC). If required, PBMC can be isolated from an individual in sufficient quantifies, allowing typing of said cells according to a method of the invention. The cells from said tissue sample can be dissociated, if required, to obtain a preferably single cell suspension. Methods for dissociation of cells, employing for example proteases such as collagenase and trypsin, are known by a skilled artisan. An RNA sample can be obtained from said cell isolate by known methods including, for example, Trizol (Invitrogen; Carlsbad, California), RNAqueous® Technology (Qiagen; Venlo, the Netherlands), Maxwell^{™} 16 Total RNA Purification Kit (Promega; Madison, Wisconsin) and, preferably, PAXgene™ Blood RNA Kit (Qiagen; Venlo, the Netherlands).

A particularly preferred tissue sample is whole blood comprising blood cells.

If required, said cell isolate can be stored prior to providing a RNA sample from said cell isolated, under conditions that preserve the quality of the RNA. Examples of such preservative conditions are known in the art and include fixation using e.g. formaline, the use of RNase inhibitors such as RNAsin™ (Pharmingen) or RNAsecure™ (Ambion), the use of preservative solutions such as RNAlater™ (Ambion), and reagents such as guananidine thiocyanate, or comparable reagents, for example, as used in the PAXgene™ Blood RNA Kit.

A cell isolate can be contacted with a stimulus by incubating said cell isolate, preferably comprising single cells, with said stimulus. For this, said cell isolate can be incubated or cultured in a medium suited for said cell isolate under conditions that favor survival of the cells. Said contacting can be for a predetermined period of time, ranging from between about 1 minute to several days. A preferred time period ranges between about 1 hour and about 24 hours, more preferred between about 2 hours and about 12 hours, and more preferred is about 5 hours. Methods and means for incubating or culturing a cell isolate are known in the art and can be obtained from, for example, Sigma-Aldrich and Invitrogen-Gibco.

Methods for determining RNA levels for a set of genes are known in the art and comprise Northern blotting, amplification methods such as based on polymerase chain reaction (PCR) and nucleic acid sequence based amplification (NASBA), and array-based methods. Preferred PCR-based methods comprise multiplex PCR and multiplex ligation-dependent probe amplification (Eldering et al., (2003) Nucleic Acids Res. 31: e153). A more preferred method is real-time quantitative PCR (Bustin (2002) J Mol Endocrin 29: 23-39).

An array format is particularly useful for this purpose. An array comprises probes specific for a gene or gene product in an arrayed format on a solid support. Said probes comprise nucleic acid molecules or mimics thereof such as peptide nucleic acid (PNA) that can hybridize to a labeled copy of RNA isolated from a cell isolate. Said probes preferably comprise a stretch of at least 20 nucleic acid residues that are identical to, or at least 95% similar to, a stretch of nucleic acid residues on a RNA molecule of which the level is to be determined, allowing base-pairing between said probe and said RNA molecule or a labeled copy thereof.

Methods for direct of indirect labeling RNA are known in the art and include Fluorescent Direct Label Kit (Agilent Technologies), GeneBeam™ First Strand cDNA Labeling kit (Enzo Life Sciences), and SuperScript™ Direct or Indirect cDNA Labeling Module (Invitrogen). The label preferably comprises fluorescent label such as cyanine 3 and cyanine 5. In a preferred embodiment, said first and second RNA sample are labeled with different dyes, allowing simultaneous hybridization of the labeled samples to a single array. Suitable hybridization and washing conditions as described in, for example, protocols from Agilent and Affymetrix can be applied for hybridization and washing of the arrays. A confocal scanning device is used to determine the intensity of label that remained associated with a probe, as a measure for the level of RNA present in a cell isolate. Different bioinformatic software tools, such as, for example, Agilent Feature Extraction, Limma ®, Edwards, Loess, and Aquantile, can be applied to analyze the data. Data analysis comprises normalization of the data to reduce bias within and between experiments, such as dye switch and dye swap (see, for example, Sterrenburg et al., Nucleic Acids Res. 2002 Nov 1;30(21):e116).

A depressive disorder refers to a disorder causing consistent loss of interest or pleasure in daily activities for at least a 2 week period, and affecting social, occupational, educational or other important functioning. Said depressive disorder can be dysthymia, a bipolar disorder or manic-depressive illness, or, preferably, major depression which is also known as clinical depression, unipolar depression, and major depressive disorder (MDD). A method of the invention can also be applied for typing a cell isolate of an individual suffering from: 1) MDD or subtypes of MDD comprising melancholic depression, atypical depression, double depression, and MDD with anger attacks; or 2) anxiety disorders (i.e panic disorder, generalized anxiety disorder, phobia, post-traumatic stress disorder, obsessive-compulsive disorder,

The genes listed in Table 1 were identified because probes specific for these genes yielded a low p-value (t-test for MDD versus Control, <0.005), a low false discovery rate (FDR) of less than 0.01%, and yielded a large effect size (>30%, M-data; >30% R-data. The FDR of a set of predictions is the expected percentage of false predictions in the set of predictions.

It has been found that the inclusion at least two genes of Table 1 in a method of the invention already provides a good prediction of the sample as being derived from an individual suffering from depression or not. A more accurate prediction is possible by including more genes of Table 1 in a method of the invention. Preferably at least three of the genes listed in Table 1, more preferred at least four of the genes listed in Table 1, more preferred at least five of the genes listed in Table 1, more preferred at least six of the genes listed in Table 1, more preferred at least seven of the genes listed in Table 1, more preferred at least eight of the genes listed in Table 1, more preferred at least nine of the genes listed in Table 1, more preferred at least ten of the genes listed in Table 1, more preferred at least eleven of the genes listed in Table 1, more preferred at least twelve of the genes listed in Table 1, more preferred at least thirteen of the genes listed in Table 1, more preferred at least fourteen of the genes listed in Table 1, more preferred at least fifteen of the genes listed in Table 1, more preferred at least sixteen of the genes listed in Table 1, more preferred at least seventeen of the genes listed in Table 1, more preferred at least eighteen of the genes listed in Table 1, more preferred at least nineteen of the genes listed in Table 1, more preferred at least twenty of the genes listed in Table 1, more preferred at least twenty-one of the genes listed in Table 1, more preferred at least twenty-two of the genes listed in Table 1, more preferred at least thirty of the genes listed in Table 1, more preferred at least fifty of the genes listed in Table 1,most preferred all of the genes listed in Table 1 are included in a method of the invention.

In a preferred embodiment, a method according the invention comprises at least two of the genes listed in Table 1, whereby said genes are numbered 1 and 2. An even more preferred method according to the invention comprises the genes numbered 1-6 in Table 1, more preferred the genes numbered 1-8 in Table 1, more preferred the genes numbered 1-12 in Table 1, more preferred the genes numbered 1-13 in Table 1 (HBG1, KRT23, AL833005, Calprin, CENTD3, PROK2, ZBTB16, F11R, FANCE, LOC150166, TMEM4, SLC7A7, and MLC1), more preferred the genes numbered 1-22 in Table 1.

Typing of a cell isolate of an individual exhibiting symptoms of depression with a method of the invention can be used to determine whether said individual is indeed suffering from a depressive disorder or is suffering from depressive symptoms that will not turn into a depressive disorder. A method of the invention can also be used to determine the biological severity of the depressive disorder. In a preferred embodiment, said typing allows prognosticating the severity of the syndrome, and/or prognosticating a response to medical treatment.

Medical treatment comprises antidepressant medication such as lithium, tricyclic antidepressants (TCAs), monoamine oxidase inhibitor (MAOIs), and selective serotonin reuptake inhibitors such as citalopram (Celexa), fluoxetine (Prozac), paroxetine (Paxil), and sertraline (Zoloft). A method of the invention is preferably used to prognosticate a response of an individual towards treatment with a selective serotonin reuptake inhibitor.

The invention further provides a set of probes for typing a cell isolate of an individual suffering from a depressive disorder or suspected of suffering there from, whereby said set of probes comprises nucleic acid sequences specific for at least two of the genes listed in Table 1. Said set of probes preferably comprises at least two probes specific for genes listed in Table 1, wherein each of said at least two probes is specific for a different gene of Table 1. Preferably said set of probes comprises at least three probes that are specific for different genes listed in Table 1. Said set of probes preferably comprises probes that are specific for each of the genes listed in Table 1. Said probes are preferably selected to hybridize to specific exons of the genes listed in Table 1, or to hybridize to the 3' ends of messenger RNA corresponding to at least two of the genes listed in Table 1. Methods for designing said probes are known in the art and have been discussed, for example, in Bouwman et al. (2006) J Neurochem 99: 84-96; and Stam et al. (2007) Eur. J. Neurosci:In press).

In a preferred embodiment, said set of probes is capable of hybridizing to at least two of the genes listed in Table 1, and/or an RNA product thereof. Said set of probes preferably comprises between 2 and 500 different probes, wherein at least two of said probes are specific for a different gene of Table 1. Preferably said set comprises between 10 and 100 different probes wherein at least two of said probes are specific for a different gene of Table 1.
In further preferred embodiment, said set of probes comprises 13 probes, wherein said set of probes is specific for the genes numbered 1-13 as listed in Table 1. The RNA levels of the genes numbered 1-13 in Table 1, HBG1, KRT23, AL833005, Calprin, CENTD3, PROK2, ZBTB16, F11R, FANCE, LOC150166, TMEM4, SLC7A7, MLC1, are preferably used for typing a cell isolate of an individual suffering from a depressive disorder.

In a particularly preferred embodiment said set comprises 22 probes, wherein each of said probes is specific for a different gene listed in Table 1.

The invention also provides the use of the set of probes according to the invention for prognosticating syndrome severity, and/or a response to medical treatment for an individual suffering from a depressive disorder.

In another embodiment, the invention provides a set of primers for typing a cell isolate of an individual suffering from a depressive disorder or suspected of suffering there from, whereby said set of primers comprises primers specific for at least two of the genes listed in Table 1. It is preferred that primers specific for a gene of Table 1 can be used in an amplification method to determine a level of expression of the said gene, Therefore, it is preferred that these primer result in the amplification of not more than 2 kilobases of a continuous stretch of nucleic acid sequences on a mRNA product of said gene, more preferred not more that 1 kilobase, most preferred between 50 bases and 200 bases. It is furthermore preferred that said stretch of nucleic acid sequences on a mRNA product span an exon-intron boundary in said gene.

The invention furthermore provides the use of the set of primers according to the invention for prognosticating syndrome severity, and/or a response to medical treatment for an individual suffering from a depressive disorder.

### Examples

### Example 1

An unchallenged sample was taken within 1 h after blood has been withdrawn (and minimally 10 min after). For this, a full heparin tube was inverted 5 times prior to opening the tube. Then, 2.5 ml was transferred to a PAXtube (PreAnalytiX GmbH) by decantation or pipeting (large tip opening). The PAXgene tube was inverted at least 10 times, and kept at room temperature for minimally 2 hours. Then, it was put at -20° C.
A challenged sample was made from the remaining blood in the heparin tube (allowing enough oxygen supply). To the remaining blood 1% (V/V) of LPS (final concentration: 10 ng LPS/ml blood) was added, and the tube was inverted 3 times to mix well immediately after addition of the LPS solution. The tube was kept at 37° C slowly rotating (or otherwise lying flat slowly shaking) for 5-6 h. After this, the tube was inverted 5 times prior to opening the tube. Then, 2.5 ml was transferred to a PAXtube. This PAXgene tube was inverted at least 10 times, and kept at room temperature for minimally 2 h. Then, it was put at -20° C.
RNA isolation proceeds according to the PAX gene protocol (PreAnalytiX GmbH). After elution the RNA concentration was checked. Samples were subsequently precipitated with 0.3 M NaAc and ethanol with the addition of 0.1 µg linear acrylamide (co-precpitant), and stored at -80°C until use. Labeling of RNA (1 µg) to use in microarray experiments was according to the Agilent protocol. Labeled RNA (1.5 µg Cy3-labeled basal sample; 1.25 µg Cy-5 labeled LPS sample) was hybridized onto the 44k Human Agilent whole genome arrays according to the protocol. After washing (Agilent protocol), the arrays were quickly dried using acetonitrile. The array was scanned using the Agilent scanner.
Signal intensities were extracted using Agilent Feature Extraction (v8.0), and the data were analyzed using Limma (R), using median signals and median background. Non-uniform signals were flagged prior to analysis. After background subtraction (Edwards, offset 30), within normalization (Loess) and between array normalization (Aquantile) data were exported to SPSS or Excel. Further data selection consisted of the following criteria (in that order), resulting in ∼25,000 genes:
- For any flagged red or green signal, the signal was discarded
- Signal intensities for any gene should be > 6.8 (log2)
- For any gene the signal should be present in >80% of the control and in >80% of the MDD samples Subsequent analysis were done on the ratio (LPS vs basal; M), the red (LPS; R) or the green (basal; G) signal.

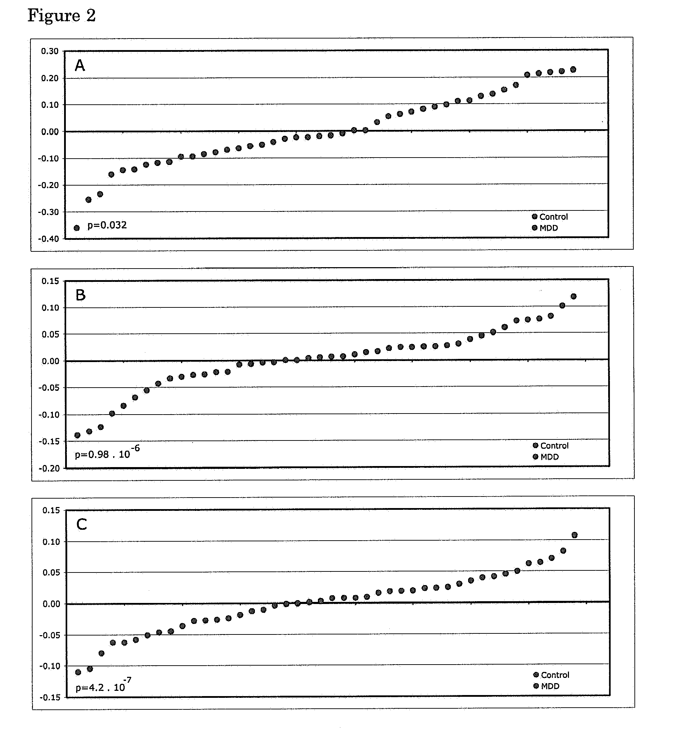

To identify classifier genes, the program PAM was used. Disease-state was used as identifier both for the M-data and the separate R- and G-dataset. The prediction errors were low, i.e. 35-40%. The analysis resulted in sets of 105 probes that participated >90% in the prediction. From these genes a selection was made based on the fact whether probes yielded a low p-value (t-test for MDD versus Control, <0.005 & FDR<0.01%), a large effect size (>30%), and whether multiple probe sets that represent a single gene had low p-values (<0.05) and high effect size (>20%) as well. In addition, the selected probe should be identifiable as an entry in Entrez Gene. As such, an initial selection of 12 genes was made. Some genes participated were informative for both the G- and R-data. For the G- and R- data, as well as for the M-data, the Pearson correlation of the expression of each individual to the average of the expression from Controls was subtracted from the Pearson correlation of the expression of each individual to the average of the expression from MDD. Samples with a high score fulfil to the average MDD profile whereas samples with a low score fulfil to the average control profile. Figures representing these correlations for the separate R-data and M-data are shown (Fig 2A,B, respectively). When the individual scores for the M-data and the G-& R-data were added, an even more robust difference could be made (Fig. 2C).

Legends to the figures

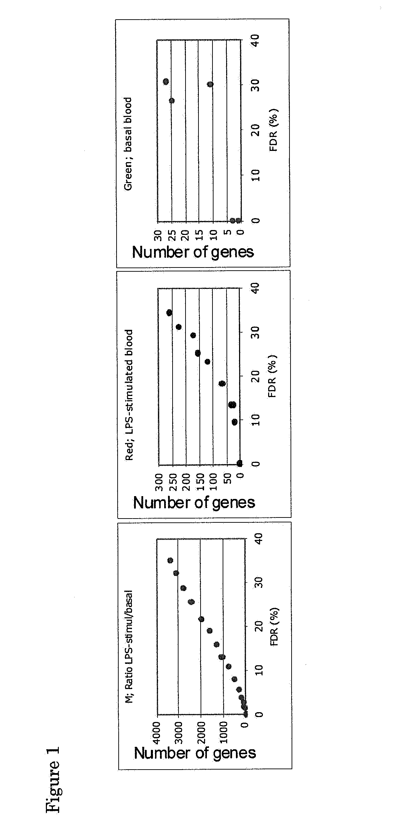

Figure 1. Scatterplots of False Discovery Rates (FDR%) to the number of genes detected for the ratio (blue), the LPS-induced signal (red) and the baseline signal (green).

Figure 2. Pearson correlations to fit the MDD or control profile for 12 classifier genes. When the correlation is positive the profile of an individual resembles that of the mean MDD profile, when negative it fits with the mean control profile. These profiles were as yet generated for 20 MDD patients and 21 healthy controls displayed in the order of the Pearson correlation. Pearson correlations for 5 genes of the LPS-regulated ratio (corrected for base-line; A), for 7 genes of the LPS-signal (B), and a summation of Pearson correlation generated by the basal signal (2 genes), the LPS-signal (7 genes), and the LPS-regulated ratio (5 genes) (C).

### References

Artigas F, Romero L, de Montigny C, Blier P (1996). Acceleration of the effect of selected antidepressant drugs in major depression by 5-HT1A antagonists. Trends Neurosci 19(9): 378-83.
Azmitia EC, Segal M (1978). An autoradiographic analysis of the differential ascending projections of the dorsal and median raphe nuclei in the rat. J Comp Neurol 179: 641-668.
Blier P, Bergeron R (1998). The use of pindolol to potentiate antidepressant meditatiën. J Clin Psychiatry 59 Suppl 5: 16-23; discussion 24-5.
Bouwman J, Spijker S, Schut D, Wächtler B, Ylstra B, Smit AB, Verhage M. Reduced expression of neuropeptide genes in a genome-wide screen of a secretion-deficient mouse. J Neurochem. 2006 Oct;99(1):84-96.
Caspi A, Sugden K, Moffitt TE, Taylor A, Craig IW, Harrington H, McClay J, Mill J, Martin J, Braithwaite A, Poulton R (2003). Influence of life stress on depression: moderation by a polymorphism in the 5HTT gene. Science 301: 386-9.
Celada P, Puig MV, Casanovas JM, Guillazo G, Artigas F (2001). Control of dorsal raphe serotonergic neurons by the medial prefrontal cortex: Involvement of serotonin-1A, GABA(A), and glutamate receptors J Neurosci 21(24): 9917-29.
Drevets WC (2000). Neuroimaging studies of mood disorders. Biol Psychiatry 48(8): 813-29.
Drevets WC, Price JL, Simpson JR, Todd RD, Reich T, Vannier M, Raichle ME (1997). Subgenual prefrontal cortex abnormalities in mood disorders. Nature 386: 824-827.
Flint J, Corley R, DeFries JC, Fulker DW, Gray JA, Miller S, Collins AC (1995). A simple genetic basis for a complex psychological trait in laboratory mice. Science 269: 1432-1435.
Fuster JM (1997). In: The prefrontal cortex. Anatomy, physiology and neuropsychology of the frontal lobe. New York: Lippincott-Raven.
Gross C, Zhuang X, Stark K, Ramboz S, Oosting R, Kirby L, Santarelli L, Beck S, Hen R (2002). Serotonin1A receptor acts during development to establish normal anxiety-like behaviour in the adult. Nature 416(6879): 396-400.
Haddjeri N, Blier P, de Montigny C (1998). Long-term antidepressant treatments result in a tonic activation of forebrain 5-HT1A receptors. J Neurosci 18(23): 10150-6.
Hajós M, Richards CD, Szekely AD, Sharp T (1998). An electrophysiological and neuroanatomical study of the medial prefrontal cortical projection to the midbrain raphe nuclei in the rat. Neuroscience 87: 95108.
Hettema JM, Neale MC, Kendler KS. A review and meta-analysis of the genetic epidemiology of anxiety disorders. Am J Psychiatry. 2001 Oct;158(10):1568-78.
Holsboer F, Lauer CJ, Schreiber W, Krieg JC (1995). Altered hypothalamic-pituitary-adrenocortical regulation in healthy subjects at high familial risk for affective disorders. Neuroendocrinology 62: 340-7.
Inder WJ, Prickett TC, Mulder RT, Donald RA, Joyce PR (2001). Reduction in basal afternoon plasma ACTH during early treatment of depression with fluoxetine. Psychopharmacology (Berl) 156(1): 73-8.
Jakab RL, Goldman-Rakic PS (2000). Segregation of serotonin 5-HT2A and 5-HT3 receptors in inhibitory circuits of the primate cerebral cortex. J Comp Neurol 417(3): 337-48.
Jorgensen H, Kjaer A, Knigge U, Moller M, Warberg J (2003). Serotonin stimulates hypothalamic mRNA expression and local release of neurohypophysial peptides. J Neuroendocrinol 15(6): 564-71.
Kagamiishi Y, Yamamoto T, Watanabe S (2003). Hippocampal serotonergic system is involved in anxietylike behavior induced by corticotropin-releasing factor. Brain Res 991(1-2): 212-21.
Kia HK, Miquel MC, Brisorgueil MJ, Daval G, Riad M, El Mestikawy S, Hamon M, Vergé D (1996). Immunocytochemical localization of serotonin(1A) receptors in the rat central nervous system. J Comp Neurol 365: 289-305.
Kirschbaum C, Hellhammer DH (1989). Salivary cortisol in psychobiological research: an overview. Neuropsychobiology 22(3): 150-69.
Kunzel HE, Zobel AW, Nickel T, Ackl N, Uhr M, Sonntag A, Ising M, Holsboer F (2003). Treatment of depression with the CRH-1-receptor antagonist R121919: endocrine changes and side effects. J Psychiatr Res 37(6): 525-33.
Mann JJ, Huang YY, Underwood MD, Kassir SA, Oppenheim S, Kelly TM, Dwork AJ, Arango V (2000). A serotonin transporter gene promoter polymorphism (5-HTTLPR) and prefrontal cortical binding in major depression and suicide. Arch Gen Psychiatry 57(8): 729-38.
McGuire MT, Troisi A (1998). Prevalence differences in depression among males and females: are there evolutionary explanations? Br J Med Psychol 71: 479-91. Merikangas K (2002). Genetic epidemiology: bringing genetics to the population-the NAPE lecture 2001. Acta Psych Scand 105: 3-13.
Montgomery AJ, Bench CJ, Young AH, Hammers A, Gunn RN, Bhagwagar Z, Grasby PM (2001). PET measurement of the influence of corticosteroids on serotonin-1A receptor number. Biol Psychiatry 50(9): 668-76.
Neumeister A, Konstantinidis A, Stastny J, Schwarz MJ, Vitouch O, Willeit M, Praschak-Rieder N, Zach J, de Zwaan M, Bondy B, Ackenheil M, Kasper S (2002). Association between serotonin transporter gene promoter polymorphism (5-HTTLPR) and behavioral responses to tryptophan depletion in healthy women with and without family history of depression. Arch Gen Psychiatry 59(7): 613-20.
Oshima A, Flachskamm C, Reul JM, Holsboer F, Linthorst AC (2003). Altered serotonergic neurotransmission but normal hypothalamic-pituitary-adrenocortical axis activity in mice chronically treated with the corticotropin-releasing hormone receptor type 1 antagonist NBI30775. Neuropsychopharmacology 28(12): 2148-59.
Peyron C, Luppi PH, Kitahama K, Fort P, Hermann DM, Jouvet M (1995). Origin of the dopaminergic innervation of the rat dorsal raphe nucleus. NeuroReport 6: 2527-2531.
Pompeiano M, Palacios JM, Mengod G (1994). Distribution of the serotonin 5-HT2 receptor family messenger-RNAs -comparison between 5-HT(2A) and 5-HT(2C) receptors. Mol Brain Res 23: 163178.
Raadsheer FC, van Heerikhuize JJ, Lucassen PJ, Hoogendijk WJ, Tilders FJ, Swaab DF (1995). Corticotropin-releasing hormone mRNA levels in the paraventricular nucleus of patients with Alzheimer's disease and depression. Am J Psychiatry 152(9): 1372-6.
Sargent PA, Kjaer KH, Bench CJ, Rabiner EA, Messa C, Meyer J, Gunn RN, Grasby PM, Cowen PJ (2000). Brain serotonin1A receptor binding measured by positron emission tomography with [11C]WAY-100635: effects of depression and antidepressant treatment. Arch Gen Psychiatry 57(2): 174-80.
Soares JC, Mann JJ (1997). The functional neuroanatomy of mood disorders. J Psychiatr Res 31 (4): 393-432.
Spijker, J, de Graaf, R, Bijl, RV, Beekman, ATF, Ormel, J, Nolen, WA (2002). Duration of major depressive episodes in the general population. Results from the Netherlands Mental Health Survey and Incidence Study (NEMESIS). Br J Psychiatry 181: 208-13.
Stahl, SM (2000). Essential psychopharmacology: neuroscientific basis and practical application. Cambridge University Press, Cambridge, UK.
Stam FJ, Macgillavry HD, Armstrong NJ, de Gunst MC, Zhang Y, van Kesteren RE, Smit AB, Verhaagen J. Identification of candidate transcriptional modulators involved in successful regeneration after nerve injury. Eur J Neurosci. 2007 Jun;25(12):3629-363.
Sterrenburg E, Turk R, Boer JM, van Ommen GB, den Dunnen JT. A common reference for cDNA microarray hybridizations. Nucleic Acids Res. 2002 Nov 1;30(21):e116.
Stockmeier CA, Shapiro LA, Dilley GE, Kolli TN, Friedman L, Rajkowska G (1998). Increase in serotonin1A autoreceptors in the midbrain of suicide victims with major depression-postmortem evidence for decreased serotonin activity. J Neurosci 18(18): 7394-401.
Sullivan PF, Neale MC, Kendler KS (2000). The genetic epidemiology of major depression: review and meta-analysis. Am J Psychiatry 157: 1552-1562.
Summers CH, Kampshoff JL, Ronan PJ, Lowry CA, Prestbo AA, Korzan WJ, Renner KJ (2003). Monoaminergic activity in subregions of raphe nuclei elicited by prior stress and the neuropeptide corticotropin-releasing factor. J Neuroendocrinol 15(12): 1122-33.

**Table 1. Gene list, indicating gene symbol or gene bank accession number, the alternative identity, and the probeIDs on the Agilent array that were used.**

| **Gene No** | **Genesymbol or Gene ID** | **Alternative ID** | **ProbeID** |
|---|---|---|---|
| 1 | HBG1 | | A_23_P53137, A_23_P64539, A_24_P289709 |
| 2 | KRT23 | | A_23_P78248 |
| 3 | AL833005 | | A_32_P164917,A_32_P164916 |
| 4 | Calprin | M11S1 | A_32_P235872, A_23_P98722 |
| 5 | CENTD3 | | A_23_P167389 |
| 6 | PROK2 | | A_24_P97342 |
| 7 | ZBTB16 | | A_23_P104804 |
| 8 | F11R | | A_24_P319364, A_24_P319369 |
| 9 | FANCE | | A_23_P42335 |
| 10 | LOC150166 | | A_32_P103815 |
| 11 | TMEM4 | | A_23_P53288 |
| 12 | SLC7A7 | | A_23_P99642 |
| 13 | MLC1 | | A_23_P211680 |
| 14 | CLEC4A | | A_23_P48029 |
| 15 | CIAS1 | | A_23_P9883 |
| 16 | RNPC1 | | A_24_P14485,A_23_P17430 |
| 17 | CORO1A | | A_23_P106761 |
| 18 | AMPD3 | | A_23_P116286, A_24_P304154 |
| 19 | ARHGEF10L | | A_23_P386, A_23_P382 |
| 20 | PRPSAP1 | | A_23_P15305 |
| 21 | COMMA8 | | A_23_P44257 |
| 22 | LMNA | | A_23_P34835, A_24_P162718 |
| 23 | AK126405 | | A_24_P766716 |
| 24 | BC035647 | | A_23_P373126 |
| 25 | BI836739 | | A_32_P194704 |
| 26 | BRI3 | | A_23_P122915 |
| 27 | C10orf125 | | A_23_P97952 |
| 28 | C16orf30 | | A_23_P37685 |
| 29 | C20orf103 | | A_23_P40295 |
| 30 | C22orf9 | | A_32_P88479 |
| 31 | CARD12 | | A_23_P119835 |
| 32 | CD163 | | A_23_P33723 |
| 33 | CDK4 | | A_23_P24997 |
| 34 | CEACAM4 | | A_24_P70480 |
| 35 | CEBPB | | A_23_P411296 |
| 36 | CHI3L1 | | A_23_P137665 |
| 37 | CKLFSF3 | | A_23_P88865 |
| 38 | CLDN5 | | A_23_P6321 |
| 39 | CR616528 | | A_24_P410686 |
| 40 | CR617678 | | A_24_P346368 |
| 41 | C9orf69 | CR602592 | A_23_P398372 |
| 42 | CXCL5 | | A_24_P277367 |
| 43 | DHRS3 | | A_23_P33759 |
| 44 | DOK1 | | A_23_P5601 |
| 45 | DOT1L | | A_23_P408768 |
| 46 | EFNB1 | | A_24_P365807 |
| 47 | EHD1 | | A_23_P52647 |
| 48 | FLJ31306 | ENST00000316535 | A_24_P495122 |
| 49 | FAM108C1 | ENST00000258884 | A_32_P39093 |
| 50 | HSPC159 | ENST00000238875 | A_23_P210330 |
| 51 | FAM13A1 | | A_23_P370651 |
| 52 | FAM78A | | A_23_P314250 |
| | | | A_23_P157875, A_23_P157879, |
| 53 | FCN1 | | A_24_P263793 |
| 54 | FCN2 | | A_23_P501732 |
| 55 | C17orf59 | FLJ20014 | A_23_P152955 |
| 56 | FLJ20035 | | A_24_P334361, A_23_P41470 |
| 57 | PID1 | FLJ20701 | A_23_P21485 |
| 58 | FU23556 | | A_23_P149798 |
| 59 | GIT2 | | A_23_P335848 |
| 60 | GLG1 | | A_23_P206510 |
| 61 | GMPR2 | | A_24_P56467 |
| 62 | GRASP | | A_23_P105442 |
| 63 | HLA-DMB | | A_32_P351968 |
| 64 | ICAM2 | | A_23_P152655 |
| 65 | ID2 | | A_23_P143143 |
| 66 | KCNE3 | | A_23_P24948 |
| 67 | KIAA1666 | | A_23_P154962 |
| 68 | KIAA1949 | | A_23_P331479 |
| 69 | KIAA2013 | | A_23_P51346 |
| 70 | LAT2 | | A_23_P259621 |
| 71 | LFNG | | A_23_P8452 |
| 72 | LIMS3 | | A_23_P365685 |
| | | | A_23_P254415, A_24_P508410, |
| 73 | LOC388524 | | A_32_P219657 |
| 74 | LOC391706 | | A_24_P675947 |
| 75 | LOC441630 | | A_24_P101960 |
| 76 | MAFB | | A_23_P17345 |
| 77 | MGC4251 | | A_23_P15516 |
| 78 | MRPS34 | | A_23_P163496 |
| 79 | NDRG2 | | A_23_P37205 |
| 80 | NEURL | | A_23_P322562, A_23_P138492 |
| 81 | PF4 | | A_24_P79403 |
| 82 | PLSCR1 | | A_23_P69109 |
| 83 | PLXNB2 | | A_24_P70888, A_32_P48397 |
| 84 | PPIF | | A_23_P202104 |
| 85 | PTP4A2 | | A_23_P23114 |
| 86 | PYDC1 | | A_23_P407614 |
| 87 | PYG02 | | A_23_P411953 |
| 88 | RAB37 | | A_23_P414654 |
| 89 | RFP | | A_32_P137035 |
| 90 | RPL26 | | A_23_P33045, A_32_P93782 |
| 91 | RPL3 | | A_32_P209350, A_23_P68942 |
| 92 | RPL34 | | A_24_P203909,A_24_P303118,A_23_P7221 |
| 93 | RPL39 | | A_23_P34018, A_32_P220307 |
| 94 | RPS17 | | A_24_P418418, A_23_P117721 |
| 95 | RXRA | | A_23_P423197 |
| 96 | SNFT | | A_23_P160720 |
| 97 | SOX4 | | A_23_P82169 |
| 98 | SPG21 | | A_23_P147450 |
| 99 | SPON2 | | A_23_P121533 |
| 100 | STAT4 | | A_23_P305198 |
| 101 | TAF12 | | A_23_P63178 |
| 102 | TNFAIP8L2 | | A_23_P46356 |
| 103 | TRAF3IP3 | | A_23_P334414, A_23_P323761 |
| 104 | TSEN34 | | A_23_P130626 |
| 105 | VAMP8 | | A_23_P28434 |
| 106 | ZDHHC7 | | A_23_P129389, A_24_P373297 |

## Claims

1. A method for typing a cell isolate of an individual suffering from a pychiatric disorder, or at risk for suffering there from, the method comprising
a. providing an RNA sample from a cell isolate from said individual;
b. determining RNA levels for a set of genes in said RNA sample, wherein said set of genes comprises at least two of the genes listed in Table 1; and
c. typing said isolate on the basis of the levels of RNA determined for said set of genes.

2. Method according to claim 1, comprising providing an RNA sample from said cell isolate after contacting said cell isolate with a stimulus.

3. Method according to claim 1 or 2, whereby said typing is based on a comparison with a reference.

4. Method according to claim 3, whereby said reference comprises RNA levels determined for said set of genes in a cell isolate prior to and/or after contacting said cell isolate with a stimulus.

5. Method according to claim 4, whereby said typing is based on the ratio of RNA levels determined in said RNA samples of a cell isolate prior to and after contacting said cell isolate with a stimulus.

6. Method according to any of the previous claims, whereby said pychiatric disorder comprises a depressive disorder.

7. Method according to any of claims 1-6, whereby said cell isolate comprises whole blood cells.

8. Method according to any of the previous claims, whereby said stimulus is selected from a cytokine, a lipopolysaccharide (LPS), a hormone, and a neurotransmitter.

9. Method according to claim 8, whereby said stimulus is LPS.

10. Method according to any of claims 1-9, whereby said set of genes comprises HBG1, KRT23, AL833005, Calprin, CENTD3, PROK2, ZBTB16, F11R, FANCE, LOC150166, TMEM4, SLC7A7, MLC1.

11. Method according to any of claims 1-10, whereby said set of genes comprises all of the genes listed in Table 1.

12. Method according to any of the previous claims, whereby said typing allows prognosticating syndrome severity, and/or a response to medical treatment.

13. Method according to claim 12, whereby said response to medical treatment comprises response to a selective serotonin reuptake inhibitor.

14. A set of probes for typing a cell isolate of an individual suffering from a depressive disorder or suspected of suffering there from, whereby said set of probes comprises probes specific for at least two of the genes listed in Table 1.

15. Use of the set of probes according to claim 14 for prognosticating syndrome severity, and/or a response to medical treatment for an individual suffering from a depressive disorder.

16. A set of primers for typing a cell isolate of an individual suffering from a depressive disorder or suspected of suffering there from, whereby said set of primers comprises primers specific for at least two of the genes listed in Table 1.

17. Use of the set of primers according to claim 16 for prognosticating syndrome severity, and/or a response to medical treatment for an individual suffering from a depressive disorder.
